# EUROPEAN PATENT APPLICATION

(11) **EP 3 241 909 A1**
(43) Date of publication of application: **08.11.2017**
(21) Application number: 15874448.2
(22) Date of filing: 30.12.2015
(51) Int. Cl.: C12Q 1/68, G01N 33/00

(54) **PCR-RFLP-BASED METHOD FOR IDENTIFYING, AND DETERMINING THE PURITY OF PISCIRICKETTSIA SALMONIS**

(30) Priority: 30.12.2014 CL 20140575
(71) Applicant: Universidad De Chile, Santiago (CL)
(72) Inventor: PULGAR TEJO, Rodrigo Enrique, Santiago (CL); CAMBIAZO AYALA, Verónica, Santiago (CL); MANDAKOVIC SEYLER, Dinka, Santiago (CL); GLASNER VIVANCO, Benjamín Enrique, SaNTIAGO (CL); ARAVENA ESPINOZA, Pamela Beatriz, Santiago (CL)
(74) Representative: Rigamonti, Dorotea
(86) International application number: PCT/CL2015/050063
(87) International publication number: WO 2016/106465

(57) **Abstract**

The present invention refers to a rapid, sensitive, specific, reproducible and low cost method for identifying and determining purity of *Piscirickettsia salmonis* bacterium. This method is a method based on PCR-RFLP (Complete) to identify *Piscirickettsia salmonis* and to establish as well if a sample of *Piscirickettsia salmonis* (for example, a bacterial culture) is pure or contaminated with other bacteria that could cohabit with *Piscirickettsia salmonis.* In this way, this method could be set up in laboratories all over the world as a standard technology to monitor bacterial cultures and in technologies for the epidemiologic control of *Piscirickettsia salmonis.* The present invention protects as well a kit for identifying and determining purity of *Piscirickettsia salmonis.*

## Description

### Field of the Invention

The present invention refers to a rapid, sensitive, specific, reproducible and low cost method for identifying and determining *Piscirickettsia salmonis* bacterium purity. This method is a method based on PCR-RFLP (Complete) to identify *Piscirickettsia salmonis* and establish as well if a sample of *Piscirickettsia salmonis* (for example, a bacterial culture) is pure or contaminated with other bacteria that might cohabit with *Piscirickettsia salmonis.* Thus, this method could be set up in laboratories throughout the world as a standard technology to monitor bacterial cultures and as technologies for the epidemiological control of *Piscirickettsia salmonis.* The present invention also protects a kit for the identification and purity determination of *Piscirickettsia salmonis.*

### Background of the Invention

*Piscirickettsia salmonis* is the etiological agent of Salmonid Rickettsial Septicemia (SRS), a disease that presents high rates of mortality that reach 30-90% of farmed salmon in Chile. Even if there are several techniques that involve classic microbiology techniques, microscopy and methods based on PCR, none of them allow establishing, specifically, the identity and purity of *Piscirickettsia salmonis* in culture media and/or in tissue samples.

SRS is a disease that spreads across salmon farming areas, accounting for 1-20% and up to 40% of monthly mortality in the total production of farmed fish in Chile (Cvitanich J.D. et al. 1991. The Isolation of Rickettsia-like organism causing disease and mortality in Chilean salmonids and its confirmation by Koch's postulate. J. Fish Dis. 14: 121-145). Its etiological agent is *Piscirickettsia salmonis,* a gram-negative, non-mobile intracellular fish pathogen, always pleomorphic but predominantly coccoid (Fryer, J. L., Lannan, C. N., Giovannoni, S. J. & Wood, N. D. Piscirickettsia salmonis gen. nov. sp. nov., the causative agent of an epizootic disease in salmonid fishes. Int. J. Syst. Bacteriol. 42, 120-6 (1992). The disease causes important losses in the world (Mauel, M. J. & Miller, D. L. Piscirickettsiosis and piscirickettsiosis-like infections in fish: a review. Vet. Microbiol. 87, 279-89 (2002); Cvitanich J.D. et al. 1991. The Isolation of Rickettsia-like organism causing disease and mortality in Chilean salmonids and its confirmation by Koch's postulate. J. Fish Dis. 14: 121-145) and its presence has been globally informed. (Olsen et al. 1997; Rodger, H.D.; E.M. Drinan. 1993. Observation of a rickettsia-like organism in Atlantic salmon, Salmo salar, L., in Ireland. J. Fish Dis. 16: 361-369; Birrell et al. 2003; Corbeil, S., Hyatt, A. D. & Crane, M. S. J. Characterisation of an emerging rickettsia-like organism in Tasmanian farmed Atlantic salmon Salmo salar. Dis. Aquat. Organ. 64, 37-44 (2005); Cusack, R. R., Groman, D. B. & Jones, S. R. M. Rickettsial infection in farmed Atlantic salmon in eastern Canada. Can. Vet. J. 43, 435-40 (2002); Brocklebank JR, Speare DJ, Armstrong RD, Evelyn TPT (1992) Septicemia suspected to be caused by a rickettsia-like agent in farmed Atlantic salmon. Can Vet J 33: 407-408).

Thus, *Piscirickettsia salmonis* bacterium is the most important pathogen in salmon industry, with economic losses over US $200 million/year. For this reason, most of the countries with an important activity in this industry have established standards to regulate its monitoring.

The facultative and non-intracellular stringent characteristics of *Piscirickettsia salmonis* (Cvitanich J.D. et al. 1991. The Isolation of Rickettsia-like organism causing disease and mortality in Chilean salmonids and its confirmation by Koch's postulate. J. Fish Dis. 14: 121-145; F Gomez, V Henriquez, S. M. Additional evidence of the facultative intracellular nature of the fish bacterial pathogen Piscirickettsia salmonis. Arch. Med. Vet. 267, 261-267 (2009); permitted to design some nutritive culture media that allow the bacteria to grow in high concentrations after 10-12 days of growth at 18°C. However, there isn't any culture medium that permits the selective growth of *Piscirickettsia salmonis,* and contamination with other bacteria has become one of the principal problems (Figueroa et al. 2012).

Several methods have been developed to detect the pathogen presence, including quantitative PCRs (Karatas, S. et al. Real time PCR detection of Piscirickettsia salmonis from formalin-fixed paraffin-embedded tissues. J. Fish Dis. 31, 747-53 (2008)), Nested PCR (Mauel, M. J.; Giovannoni, S. J.; Fryer, J. L. 1996. Development of polymerase chain reaction assays for detection and differentiation of Piscirickettsia salmonis. Dis. Aquat. Org. 26: 189-195) and an indirect fluorescence antibody test (Lannan, C. N., Ewing, S. A. & Fryer, J. L. A Fluorescent Antibody Test for Detection of the Rickettsia Causing Disease in Chilean Salmonids. J. Aquat. Anim. Health 3, 229-234 (1991)) and an indirect fluorescence antibody test (IFAT) (Lannan, C. N., Ewing, S. A. & Fryer, J. L. A Fluorescent Antibody Test for Detection of the Rickettsia Causing Disease in Chilean Salmonids. J. Aquat. Anim. Health 3, 229-234 (1991)), that allow detecting *Piscirickettsia salmonis* in tissue samples and in culture media but none of them can guarantee its purity.

The principal methods currently used for the detection of *Piscirickettsia salmonis* are based on PCR and immunodetection (IFAT); however, they are not sufficiently specific and generate positive results for other fish pathogenic bacteria and environmental pollutants. For this reason, these methods do not serve for defining the ultimate cause of mortality and for assessing *Piscirickettsia salmonis* purity, a necessary characteristic for vaccine design. The method of the present invention (PCR-RFLP, Polymerase Chain Reaction-Restriction Fragment Length Polymorphism)) directly benefits aquaculture industry and the veterinary industry for the production of solutions against *Piscirickettsia salmonis* (SRS) infection.

Various methods are described in the prior art that use PCR and PCR-RFLP techniques for different purposes, for example, to discriminate between specific genes in marine organisms, to identify species, among others. Some of said publications are mentioned below:
Publication CN103923972 discloses a method based on PCR-RFLP that distinguishes COI gene of 17 sea cucumbers (*Stichopodidae*). Thus, starting from DNA samples of the species, a template is established and a fragment of COI gene is amplified using primers COIE-F: 5'-ATAATGATAGGAGGRTTTGG-3', COIE-R: 5-GCTCGTGTRTCTACRTCCAT-3'PCR'; then enzymatic digestion of the COI fragment is carried out using restriction endonuclease DdeI/SFCI or BstNI/Sau3AI, to conduct thereafter electrophoresis of the digestion product obtained. Afterward, the size of each of the bands resulting from electrophoresis of the samples is estimated, contrasting the number and size thereof with the reference band spectra of the seventeen sea cucumbers. The method can extensively be applied, is simple to operate, and performs an accurate and quick detection, being particularly adequate to identify a great number of sea cucumbers that provide basic products; for this reason it can assist in the standardization of the market of raw materials from sea cucumbers and in the protection of said resources.

CN103525933 discloses a PCR-RFLP method for distinguishing *Ctenopharyngodon idellus* from *Mylopharyngodon piceus,* and using specific molecular markers discloses a DNA fragment for distinguishing between these two species. The method comprises a group of primers and the amplification of a fragment of cytochrome b gen, 1140 bp in length of *Ctenopharyngodon idellus* and *Mylopharyngodon piceus.* The PCR product is subjected to enzymatic digestion by the use of restriction endonuclease BglI. The fish is *Ctenopharyngodon idellus* if two bands appear in the electrophoresis pattern, and is *Mylopharyngodon piceus* if one band appears in the electrophoresis pattern. Consequently, the species is identified accurately and quickly by the change in the restriction pattern of the amplification product

The invention of CN103436612, describes a method of PCR-RFLP for the prompt detection of common sturgeons. The method comprises DNA extraction from the genome of a sample that is then used as a template, the PCR amplification reaction is then carried out and after this the enzymatic digestion of this product is conducted using restriction enzymes. The digestion product is subjected afterwards to fragmentation by electrophoresis in agarose gel and the identification of the species is performed according to the differences observed in the electropherogram. The method provided by the invention is accurate, quick and simple to operate, allowing to identify fish fry from common sturgeons.

The invention of CN103290131 describes a pair of primers (forward and reverse) and a kit to distinguish the fish *Channa argus* and *Channa maculata.* The kit that contains the primer pair also comprises a conventional PCR reagent, Taq polymerase buffers, dNTPs (deoxyribonucleotide triphosphates), a digestion reagent, a digestion buffer and an EcoRI restriction enzyme. The method described by the invention is simple to operate, and only implies cutting a small sample of fin or muscles, to ensure the fish survival, and is rapid and accurate.

The invention in CN101724690 describes a method for detecting polymorphism in the flora of water for shrimp farming from genomic DNA of microbes mixed in a sample of the water for shrimp farming and the PCR reaction is performed designing a universal primer T-RFLP-PCR. The product is purified and the enzymatic DNA cleavage is performed by the specific restriction enzyme HaeIII, then the DNA segments are separated on a 1% agarose gel conducting the fluorescent scanning on the DNA segments. After having analysed the polymorphism structure of the microbial flora, the quantitative detection of the predominant bacteria is performed by means of fluorescence with *in situ* hybridization technology. The method is highly reproducible, sensitive, rapid, accurate and stable, and allows conducting the qualitative and quantitative analysis of the ecological diversity of microbes in a body of water for shrimp farming.

US2008305484 teaches the specific identification of a Campylobacter species for which there is an absence of adequate biochemical assays for its differentiation. Twelve species of *Campylobacter* are studied based on the partial sequence (1,020 bp) of the gyrB gene (DNA topoisomerase beta-subunit gene) and the topology of the resulting phylogenetic tree based on the gyrB gene was similar to the topology of a phylogenetic tree previously reported based on the 16S rDNA gene. However, the gyrB gene provides a better resolution than the 16S rDNA gene for *Campylobacter* species, with sequence similarities among species ranging from 58.3 to 89.2%. A set of universal primers was designed to amplify a 960 bp fragment of gyrB gene of *Campylobacter spp.,* which was used for PCR-RFLP of 19 strains that represented the twelve Campylobacter species, including C. *jejuni* subsp *jejuni*, C.*coli, C. concisus C. curves, C. showae, C. mucosalis*, *C. fetus, C. hyointestinalis, C. sputorum biovar sputorum*, *C. helveticus, C. upsaliensis and C. lari.* The enzymatic digestion of the 960 bp fragment was performed with the restriction enzyme DdeI, XspI, or the combination of HindIII and MboI as a double digestion, resulting in unique digestion patterns for the twelve *Campylobacter* species. Specific primer sets were also used to amplify regions of the gyrB gene specific for each *Campylobacter* species, generating products that ranged from 86 to 493 bp in size. It was established that the specific primer sets of *Campylobacter* species were highly specific. The method is fast and unequivocally identifies most *Campylobacter* species.

ES2161135 describes the identification of Auxis thazard in canned fish, by amplifying a 187 bp fragment (BDR) of mitochondrial cytochrome b gene DNA, further using a restriction endonuclease. This helps to differentiate it from other species used as substitutes of Auxis thazard by PCR-RFLP.

Meanwhile, the proposed solution in this case refers to a method for the amplification of a fragment of bacterial 16S ribosomal gene (16S rDNA), shared by all bacteria, through the use of universal primers, and the subsequent digestion of the amplified by means of restriction enzymes (PCR-RFLP) that recognize specific nucleotides and in particular 16S rDNA positions of *Piscirickettsia salmonis.* This digestion product can be visualized in an electrophoresis, which allows to identify the specific and characteristic digestion pattern of *Piscirickettsia salmonis.* The presence of a pattern or electrophoretic bands different from those of *Piscirickettsia salmonis* pattern indicates the presence of other bacteria in the sample. The methodology is rapid, highly specific, low cost and as sensitive or more sensitive than the detection assays currently used.

5 groups of restriction enzymes are disclosed, each group being associated to a specific digestion band pattern, i.e., 5 isoschizomer groups. According to the digestion band pattern of the restriction enzyme groups, these are as follows: Group 1: PmII, PMACI, ACVI, BbrPI, Eco72I and PspCI; Group 2: BspCNI and BseMII; Group 3: TspEI, MluCI, Sse9I, TasI and Tsp509I; Group 4: BfaI, FspBI, XspI and MaeI; and Group 5: DdeI, BstDEI and HpyF3I.

The present method allows the individual or combined use of the restriction enzymes that generate the above mentioned digestion band patterns.

The present invention has been tested under controlled laboratory conditions for microbiological cultures of *Piscirickettsia salmonis* and in samples in the field, that is, to say, in tissues of farmed fish infected with *Piscirickettsia salmonis.*

There are techniques for detecting *Piscirickettsia salmonis,* but these have low specificity, generating false positives. For purity determination in cultures or in fish tissues, classic microbiological techniques are used, such as Gram stain, but this lacks specificity for *Piscirickettsia salmonis.* The method of the present invention is distinguished from those existing for its high specificity to detect *Piscirickettsia salmonis* that avoids the generation of false positives, improving diagnostic capacity and the possibility of monitoring *Piscirickettsia salmonis* cultures to certify their purity.

The present method is a method based on PCR-RFLP to identify *Piscirickettsia salmonis* and also to establish if the sample is pure or is contaminated with other environmental pathogens or bacteria that could cohabit with *Piscirickettsia salmonis.* Thus, this method could be established in laboratories throughout the world as a standard technology to monitor bacterial cultures and in technologies for the epidemiological control of *Piscirickettsia salmonis.*

### Brief Description of the Invention

The present invention further refers to a quick, sensitive, specific, reproducible and low cost method to identify and determine the purity of *Piscirickettsia salmonis.* The present method is based on PCR-RFLP techniques to identify *Piscirickettsia salmonis* as well as to establish if the sample is pure or contaminated with other bacteria or pathogens that might cohabit with *Piscirickettsia salmonis.* The present invention also protects a kit for identifying and determining *Piscirickettsia salmonis* purity.

A comparison of the sequences of the 16S rDNA gene of *Piscirickettsia salmonis* with sequences of 16S rDNA genes of other pathogenic and environmental bacteria of the fish, as well as bacterial contaminants that grow in *Piscirickettsia salmonis* culture media, revealed the presence of specific nucleotides and in particular positions of the 16S rDNA gene of *Piscirickettsia salmonis.* This analysis was complemented with the comparison of the 16S rDNA gene sequences of all the bacteria present in public databases SILVA, RDP, Greengenes and NCBI.

After obtaining these sequences, an alignment was conducted to search for 100% preserved nucleotides in the genus *Piscirickettsia,* and afterward these nucleotides were contrasted against 16S rDNA genes of other bacteria to confirm which of these are unique to the genus. With this information we proceeded to select restriction enzymes that specifically recognize the particular sequences identified in the 16S rDNA gene of *Piscirickettsia salmonis.* After this, the selected enzymes were grouped by the cut position of the 16S gene, and then band patterns were calculated *in silico* for each enzyme group.

The enzyme selection allowed to determine the patterns that allow to distinguish the species of interest from all the other analyzed species and genera, as it is shown in the flow diagram of Figure 1, Table 1 and Figures 2 and 3.

**Table 1 Digestion patterns, cutting sequence and restriction enzyme groups.**

| **Pattern** | **Pattern 1** | **Pattern 2** | **Pattern 3** | **Pattern 4** | **Pattern 5** |
|---|---|---|---|---|---|
| Cutting sequence | CAC/GTG | TCAG (9/7) | /AATT | C/TAG | C/TNAG |
| Endonucleases or restriction enzymes associated to the pattern (isoschizomers) | PmII | BspCNI | TspEI | BfaI? | DdeI |
| | PmaCI | BseMII | MluCI | XspI | BstDEI |
| | AcvI | | Sse9I | MaeI | HpyF3I |
| | BbrPI | | TasI | | |
| | Eco72I | | Tsp509I | | |
| | PspCI | | | | |

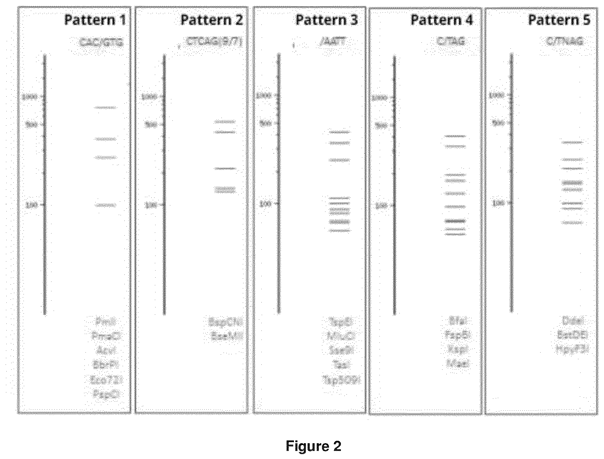

In this way, starting from genomic ADN extraction, either from infected tissue from salmonids, cell cultures infected with *Piscirickettsia salmonis* or *Piscirickettsia salmonis* growing in an artificial medium in a bacterial culture, the present method allows to carry out a polymerase chain reaction (PCR) protocol to amplify a region in the pathogen genome using universal primers (27F 5'AGA GTT TGA TCA TGG CTC AG3') and 1492R (5'CGG TTA CCT TGT TAC GAC TT3') for the amplification of the 16S rDNA gene, amplifying the version of this gene in any bacterium present in the sample. The PCR products thus obtained are enzymatically digested by an enzyme representative of the 5 restriction enzyme groups indicated in Table 1, such as PmII, which produces 3 cuts and 4 bands that may be observed in an agarose or acrylamide gel electrophoresis, see Figure 2. This characteristic pattern of *Piscirickettsia salmonis* allows to determine in 3 hours its identity and the purity of a sample.

### Brief Description of the Figures

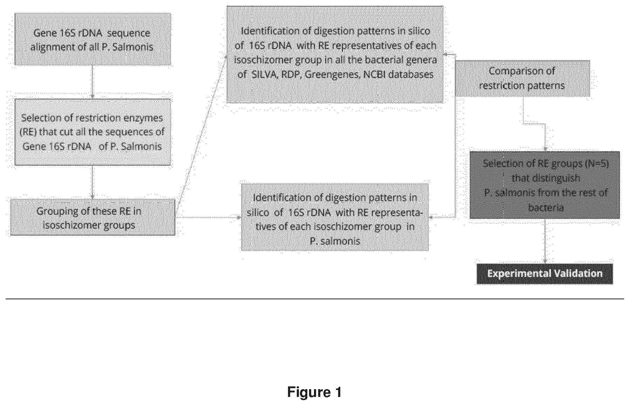

Figure 1: Represents a flow diagram of the methodology conducted to determine RFLP patterns. They are shown as consecutive steps represented as rectangles and unions in blue represent the logic order.
Figure 2: The five digestion patterns that allow to distinguish a *Piscirickettsia salmonis* from the rest of the bacteria are observed. These bacteria represent examples of the bacteria that cohabit with *Piscirickettsia salmonis* both in natural environments and in a laboratory. Each pattern is formed by digestion of the amplification product by PCR (with universal primers 27F and 1492R) of 16S ribosomal gene of *Piscirickettsia salmonis,* by means of a restriction endonuclease or an isoschizomer thereof, which are listed in blue in the lower area of each pattern. The nucleotide sequences of recognition used for digestion by restriction enzymes are also shown in red on the upper area of each pattern. Each pattern is accompanied by a size standard (with units of 100 nucleotides) that allows estimating the band sizes of each generated pattern.

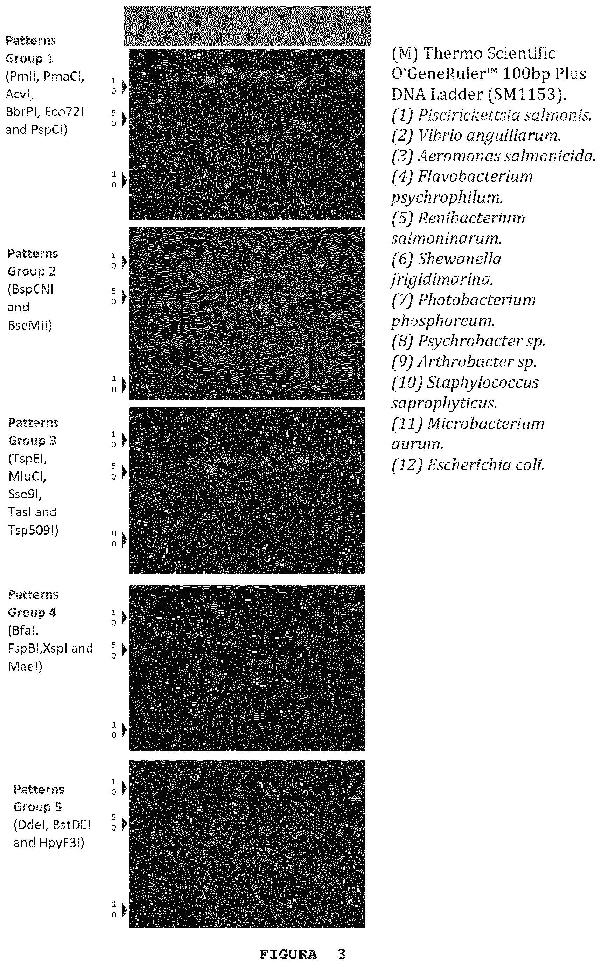

Figure 3: The experimental validation of the five digestion patterns is observed. These patterns allow to distinguish a *Piscirickettsia salmonis* from the rest of the bacteria used as an example. Each pattern is formed by digestion of the PCR amplification product (with universal primers 27F and 1492R) of 16S ribosomal gene of *Piscirickettsia salmonis and* the other relevant bacterial genera by means of a restriction endonuclease or any one of its isoschizomers in the groups indicated in Table 1, which are found listed in blue in the lower area of each pattern, to the left of each electrophoresis image.

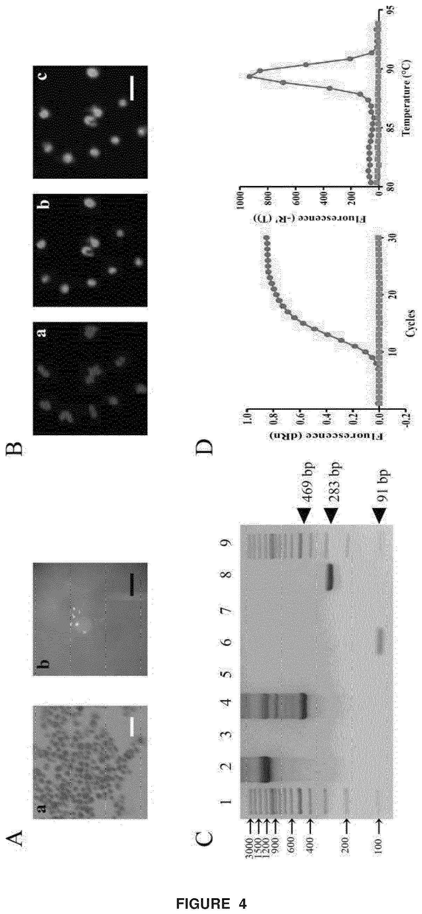

Figure 4A-4D: Characterization and detection of *Piscirickettsia salmonis* by traditional techniques. Figure 4A: Gram stain of *Piscirickettsia salmonis* cells. White bar = 1.25 µm (a) and the colony morphology. Black bar = 1.25 cm (b). Figure 4B: IFAT assay of *Piscirickettsia salmonis* cells in exponential growth observed by confocal microscopy, in blue, DAPI staining that marks genetic material (a); in green, fluorescence associated to the immunodetection antibody of *Piscirickettsia salmonis* (b) and in light blue the overlapping of above signals (c) White bar = 2.5µm. Figure 4C: 2% agarose gel electrophoresis. Figure 4D: qPCR reaction curves that use primers Ps16SRNA-F1/Ps16SRNA-R in reactions with or without *Piscirickettsia salmonis* DNA as substrate: (left) amplification curve and (right) fusion curve.

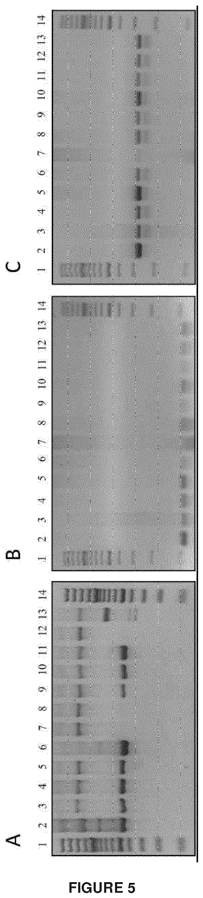

Figure 5A-5C: Nested PCR Amplification and ITS-PCR assay. 2% agarose gel electrophoresis of (A) Nested PCR amplification of ribosomal DNA of 16S (primers Ps2s/PsAs), Figure 5A, (B) ITS-PCR using primers RTS1/RTS2, Figure 5B and (C) ITS-PCR using primers RTS1/RTS4, Figure 5C: (lane 1) ladder 100 bp plus. (lane 2) *Piscirickettsia salmonis.* (lane 3) *Vibrio anguillarum;* (lane 4) *Aeromonas salmonicida;* (lane 5) *Flavobacterium psychrophilum;* (lane 6) *Renibacterium salmoninarum;* (lane 7) *Shewanella frigidimarina;* (lane 8) *Photobacterium phosphoreum;* (lane 9) *Psychrobacter sp.;* (lane 10) *Arthrobacter sp.;* (lane 11) *Staphylococcus saprophyricus;* (lane *12) Microbacterium aurum;* (lane 13) *Escherichia coli;* (lane 14) ladder 100bp plus.

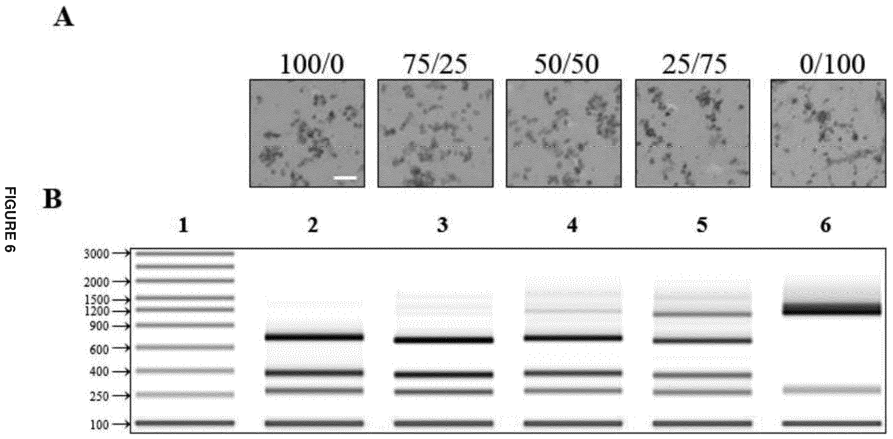

Figure 6A-6B: Assay with mixed samples. *Piscirickettsia salmonis and Vibrio anguillarum* (in the same growth stage) were mixed in different proportions: 100/0, 75/25, 50/50, 25/75 and 0/100 of *Piscirickettsia salmonis*/*Vibrio anguillarum* and subjected to Gram stain. Bar corresponds to 1.25 µm (Figure 6A). After 16S rDNA amplification of the mixed samples and digestion with restriction enzyme *PmlI,* the resulting PCR-RFLP digestion pattern was examined by electrophoresis using DNA ScreenTape in the instrument Tape Station 2200 (Figure 6B): (lane 1) ladder 100 bp plus (lane 2) 100% *Piscirickettsia salmonis*/0% *Vibrio anguillarum;* (lane 3) 75% *Piscirickettsia salmonis*/25% *Vibrio anguillarum;* (lane 4) 50% *Piscirickettsia salmons*/50% *Vibrio anguillarum;* (lane 5) 25% *Piscirickettsia salmonis*/ 75% *Vibrio anguillarum;* (lane 6) 0% *Piscirickettsia salmonis*/ 100% *Vibrio anguillarum*

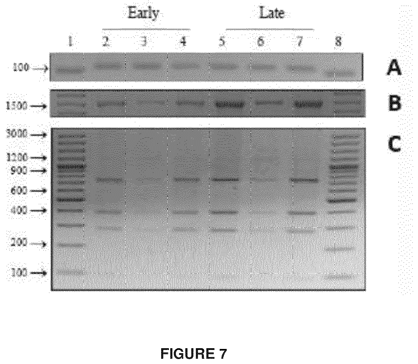

Figure 7: Assay of samples of different tissues and different stages of the infection. DNA extracted from tissues of fish infected with *Piscirickettsia salmonis* was used to amplify 16S rDNA and carry out PCR-RFLP assays. "Early" represents early stages of the infection in farmed fish that do not show symptoms of the disease. "Late" represents late stages of the infection in farmed fish that show symptoms of the disease **(A)** PCR amplification of 60S rDNA gene (Ss60sS27F/Ss60s27R) of salmon. **(B)** PCR amplification of 16S rDNA (primers 27F/1492R). **(C)** PCR-RFLP digestion pattern (16SrDNA amplification using primers 27F/1492R and digestion by restriction enzyme *PmlI*)*.* In all the cases: (lanes 1 and 8) way 100 bp plus (Thermo scientific); (lanes 2 and 5) kidney samples; (lanes 3 and 6) spleen samples (lanes 4 and 7) brain samples.

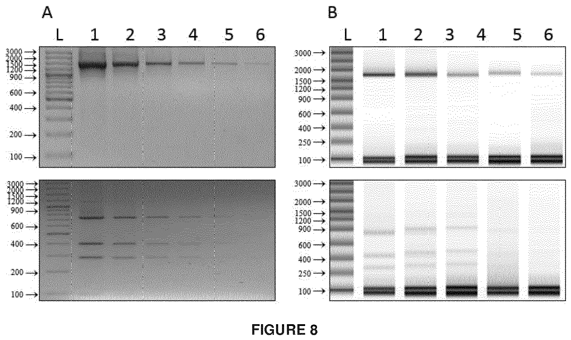

Figure 8: Sensitivity assays. On 2% agarose gels **(A),** (Top) 16S rRNA amplicons of *Piscirickettsia salmonis*: (lane 1) ladder 100 bp plus (Thermo scientific); (lane 2) 500 ng PCR product; (lane 3) 250 ng PCR product; (lane 4) 125 ng PCR product; (lane 5) 62,5 5 ng PCR product; (lane 6) 31,25 ng PCR product. Bottom). Enzymatic digestion with *PmlI* of classified PCR products correlative with panel A. In Bioanalyzer Tape Station (Agilent Technologies) **(B),** (Top) amplicons of 16S rRNA Piscirickettsia *salmoms*: (lane 1) ladder tape station (Agilent Technologies); (lane 2) 1 ng PCR product; (lane 3) 0.5 ng PCR product; (lane 4) 0.25 ng PCR product; (lane 5) 0.125 ng PCR product; (lane 6) 0.0625 ng PCR product. (Bottom). Enzymatic digestion with *PmlI* of classified PCR products correlative with panel B.

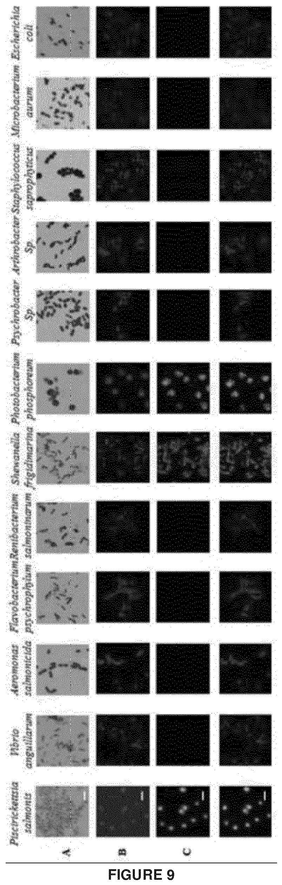

Figure 9: Gram stain and IFAT essays for P. *salmonis* and cohabiting bacteria. (A) Microphotography of bacteria after Gram stain. The white bar represents 1.25 □m. (B) Images by confocal microscopy of bacteria stained with DAPI (DNA marker) and with antibody anti-P. *salmonis* (BiosGroup). The series of lower images represent images compounded of B and C, wherein the light blue colour represents the presence of genetic material and immunodetection for each bacterium. The bar corresponds to 2.5 □m.

### Detailed Description of the Invention

The solution put forward herein refers to a method of amplification of 16S ribosomal gene shared by all the bacteria present in a sample, and the subsequent digestion of the amplified gene by restriction enzymes (PCR-RFLP) that recognize specific nucleotides and in particular positions of 16S rDNA gene of *Piscirickettsia salmonis.* This digestion product can be visualized in an electrophoresis, which allows to identify the specific and characteristic digestion pattern of *Piscirickettsia salmonis.* The presence of a pattern or electrophoretic bands different from those of *Piscirickettsia salmonis* pattern in the sample indicates the presence of other bacterium in the sample. The methodology is rapid, highly specific, low cost and as sensitive as the PCR assay currently in use. 5 groups of restriction enzymes are disclosed, each group associated to a specific band digestion pattern. The restriction enzyme groups are the following according to the band digestion pattern: Group 1: PmII, PmaCI, AcvI, BbrPI, Eco72I and PspCI; Group 2: BspCNI and BseMII; Group 3: TspEI, MluCI, Sse9I, TasI and Tsp509I; Group 4: BfaI, FspBI, XspI and MaeI; and Group 5: DdeI, BstDEI and HpyF3I.

The present method allows the individual or combined use of the restriction enzymes that generate the above mentioned band digestion patterns.

The method of the present invention is distinguished from existing methods for its high specificity in the detection of *Pisscirickettsia salmonis,* which avoids to generate false positives, improving diagnostic capacity and the possibility to monitor *Piscirickettsia salmonis* cultures in order to certify their purity, both in microbiologic media and in host cell cultures.

In this way, the present invention consists in the extraction of genomic DNA, either from tissue of an infected salmon, from a cell culture infected with *Piscirickettsia salmonis* or from *Piscirickettsia salmonis* growing in an artificial medium in a bacterial culture. After this a polymerase chain reaction (PCR) protocol is conducted in order to amplify a region from the genome of any bacterium present in the sample using universal primers 27F and 1492R (and their derivatives) that amplify a fragment of 16S ribosomal gene. The PCR product obtained in this way is enzymatically digested by an enzyme that belongs to one of the 5 groups of restriction enzymes indicated in Table 1. For example, if *Piscirickettsia salmonis* is the only bacterium present in the mixture, the enzyme PmlI produces 3 cuts and 4 bands in agarose or acrylamide gel electrophoresis, see Figure 2. This pattern is characteristic of *Piscirickettsia salmonis* and allows to determine the identity and purity of a sample in a protocol that does not take longer than 3 hours.

### Example 1: Bacteria, growth media and conditions

*Piscirickettsia salmonis* (LF-89) was cultured at 18°C under constant agitation in the liquid culture medium described by Vera and colleagues (Vera et al. 2012 http://www.scielo.cl/ scielo.php?pid=S0301-732X2012000300010&script=sci arttext).

The broth contains 17.0 g/l casein peptone, 3.0 g/l soy flour peptone, 2.5 g/l D (+) glucose, 20.0 g/l NaCl, 2.5 g/l dipotassium monohydrogen phosphate (Broth A), which was complemented with 0.1% L-Cys, 2.5% SBF, 10 mg/l FeCl₃, 15 g/l NaCl. The cultures were incubated at 17°C under gentle stirring. The broth was used to generate solid culture media, with 15 g/l agar-agar and 15 g/l NaCl, was sterilized in an autoclave and was complemented with 1 g/l L-Cys, 20 mg/l FeCl₃, 5% SBF. The cultures were incubated at 17°C for 12 days.). All the other bacteria, *Piscirickettsia salmonis, Vibrio anguillarum, Aeromonas salmonicida, Flavobacterium psychrophilum, Renibacterium salmoninarum, Shewanella frigidimarina, Photobacterium phosphoreum, Psychrobacter sp, Arthrobacter sp, Staphylococcus saprophyticus, Microbacterium aurum and Escherichia coli* were cultured under the same conditions

### Example 2: Obtainment of bacterial isolate from fish and in a laboratory environment

Tissue samples from Atlantic salmon were directly obtained from the fish cage (*Salmo salar*). The samples were taken from heart, kidneys and gills and were rubbed gently on glass plates (microscope slides), and cultured in solid growth medium for 5 days at 18°C. In addition, four bacterial environmental contaminants were obtained by exposing flasks that contained liquid culture medium to the laboratory environment. Samples of this medium were also cultured in solid medium for 5 days at 18°C. All the isolated samples were cultured in the same medium and growth conditions described above.

### Example 3: DNA Extraction and 16S rDNA amplification

Bacterial ADN was purified from 1 mL of culture in exponential growth OD₆₀₀: ∼0.5 or from infected tissue samples (≤ 20 mg) using DNeasy Blood & Tissue Quiagen kit, among others. In the case of bacterial culture samples, these were centrifuged 10 minutes at 8.000 rpm discarding the supernatant, and in the case of tissue samples the standard protocol recommended by the manufacturer was used. The 16S rDNA was amplified by PCR with universal primers 27F 5' AGA GTT TGA TCA TGG CTC AG 3' and 1492R 5' TAC GGT TAC CTT GTT ACG ACT T 3'. The PCR amplifications were conducted in 25 µL volume that contained 200 µg Bacterial DNA (∼ 4 µL) and 20 µL of an amplification cocktail that contained the following components: 12,5 µL GoTaq mix Promega, 5,5 µL nuclease-free water and 1 µL progressive or reverse primers, each at 10 mM final concentration. The PCR amplification was carried out in a cyclic thermal controller MJ research, Inc. The samples were incubated for 10 minutes at 95°C to denature DNA, the amplification was carried out in 30 cycles at 95°C for 60 seconds, 58°C for 30 seconds, and 72°C for 60 seconds. The samples were incubated then at 72°C for 10 minutes for a final extension and maintained at 4°C while waiting to be analysed. The amplified DNA was fractioned by electrophoresis in a 2% w/v agarose gel in TAE buffer. The gels were stained with ethidium bromide to detect the amplification product and were photographed. The PCR products were sequenced and bacterial identity was verified by comparison with bacterial 16S rDNA databases using BLAST algorithm.

### Example 4: Restriction analysis of the amplified 16Sr DNA

Five microliters (of each amplified PCR product) were digested with restriction endonucleases for 30 minutes at 37°C. The restriction fragments were fractioned in 2% w/v agarose gel stained with con ethidium bromide and photographed, the lengths of the restriction fragment were calculated comparing them to standard 100 bp Plus DNA Ladder, Thermo Scientific O'GeneRuler™ (SM1153).

The restriction fragments were also analysed in the Tape Station 2200, Agilent Technologies using Genomic DNA Screen Tape, Kit Agilent plus genomic DNA reagents according to the manufacturer indications.

### Example 5. Selection of restriction enzymes

The nucleotide sequence of the 16S rDNA fragment sequenced from our *Piscirickettsia salmonis* isolates was used to validate its identity and select the 16S rDNA genes of this genus in ribosomal DNA SILVA, RDP and GreenGenes databases. These sequences were subsequently aligned and the conserved and variable regions were identified comparing all the selected sequences. From this information, all the restriction enzymes that cut all the 16S rDNA sequences of *Piscirickettsia salmonis* were selected. After this, the selected restriction enzymes were used to generate cutting patterns of all the 16s rDNA sequences (from the complete ribosomal DNA in SILVA, RDP and GreenGenes databases) that were susceptible of being digested by them. This comparative analysis allowed us to identify one restriction enzyme subgroup (Table 1) that generates an exclusive digestion pattern for *Piscirickettsia salmonis,* which allows distinguishing this bacterium from all the rest of the analysed bacteria. In the experimental validation of this prediction of digestion patterns, the following bacteria were used: *Vibrio anguillarum, Aeromonas salmonicida, Flavobacterium psychrophilum, Renibacterium salmoninarum, Shewanella frigidimarina, Photobacterium phosphoreum, Psychrobacter sp, Arthrobacter sp, Staphylococcus saprophyticus, Microbacterium aurum and Escherichia coli,* from which DNA was extracted, 16S ribosomal gene was amplified with universal primers, digested with restriction enzymes of the groups indicated in Table 1 and visualised in 2% agarose gels, as previously described.

### Example 6: Quantitative Nested PCR and Taqman probe Assays:

Nested PCR tests were carried out with the method and the primers described in Mauel et al. 2006 using Ps2s 5' CTA GGA GAT GAG CCC GCG TTG 3' and PsAs 5' GCT ACA CCT GCG AAA CCA CTT 3' primers in a thermal cyclic controller of MJ Research, Inc. The samples were denatured at 95°C for 5 minutes, and afterwards, in 30 cycles conducted at 95°C for 1 minute, at 60°C for 30 seconds and at 72 °C for 2 minutes. The PCR reaction was conducted in 25 µL of final reagent that contained 2 µL of the above-described PCR product of 16S amplification, 12.5 µL GoTaq, 8.5 µL nuclease-free water and 1 µL of each primer.

The qPCR assay was carried out using 16SRNA-F15' AGG GAG ACT GCC GGT GAT A 3' and 16SRNA-R5' ACT ACG AGG CGC TTT CTC A 3' primers described by Karatas, S. et al. Real time PCR detection of *Piscirickettsia salmonis* from formalin-fixed paraffin-embedded tissues. J. Fish Dis. 31, 747-53 2008. The reaction was conducted with a final10 µL volume that contains 50 ng gDNA and each primer at 10 mM final concentration using the Light Cycler Syber Green, Roche kit. The amplifying conditions were: 10 minutes at 95°C, followed by 30 amplification cycles at 95°C for 10 seconds, at 55°C for 15 seconds, and an extension at 72°C for 10 seconds.

The assays with TaqMan probe were carried out as described in Corbeil, S., et al 2003. Development of a TaqMan quantitative PCR assay for the identification of Piscirickettsia salmonis. Bulletin of the European Association of Fish Pathologists 23:95-101. The primer used was F-760 5' TCT GGG AAG TGT GGC GAT AGA 3' and R-836 5' TCC CGA CCT ACT CTT GTT TCA TC 3' and 6-carboxyfluorescein 6FAM and 6-carboxytetramethylrhodamine TAMRA, labelling probe PS23S: 6FAM-TGA TAG CCC CGT ACA CGA AAC GGC ATA-TAMRA. All the reactions were conducted in the sane thermo cycler as other qPCR reactions. For each reaction, the primers were used in the recommended concentrations: 900 nM for each primer and 250 nM for each 23S FAM probe. Cycle conditions were the following: 50°C for 2 minutes, 95°C for 10 minutes, followed by 40 cycles at 95°C for 15 seconds and at 60°C for 1 minute.

### Example 7: Gram Staining, Fluorophore and Indirect Fluorescent Antibody Test IFAT:

Gram staining was performed affixing samples on a glass plate with heat. The staining process was carried out using violet crystal for 1 minute, iodine for 1 minute, ethanol for 30 seconds and safranin for 1 minute. Between each stage, the samples were washed with distilled water. All the samples were observed in a Nikon Eclipse Ni microscope with 100x objective with oil immersion. To mix the two bacterial cultures of *Piscirickettsia salmonis*/*Vibrio anguillarum, they* were grown to OD₆₀₀=0.5 and mixed in different proportions: 100/0, 75/25, 50/50, 25/75 and 0/100 *Piscirickettsia salmonis*/*Vibrio anguillarum.*

The IFAT test was performed according to the manufacturer's recommendations (SRS-Fuorotest indirect, GrupoBios) with some modifications, as it will be indicated below. 20 uL were left to dry at room temperature; then, the samples were fixed with dilute paraformaldehide in 4% PBS for 10 minutes, followed by three washings with PBS sterile solution.

100 uL of previously diluted Oligoclonal reactive 1:100 with dilution solution were added to each sample, and were incubated for 30 minutes at room temperature in a humid chamber. The samples were washed for four minutes with wash solution previously diluted 1:25 with distilled water. Subsequenly, 100 uL of solution anti-IgG FITC diluted 1:100 and DAPI diluted 1:200 in dilution solution were added to each sample. The plates were incubated at room temperature for 30 minutes in a humid chamber avoiding light, and afterwards, the samples were washed twice with wash solution diluted 1:25 with distilled water for four minutes.

Finally, 6 uL of Mounting Dako medium were added and placed on a glass plate, to be subsequently stored at 4°C when the mounting medium had solidified. The samples were analysed in a confocal Nikon Eclipse Ti microscope using 60X objective with immersion in oil.

### Example 8: Fish Infection and PCR-RFLP from tissues of infected fish in fish farms:

The assays were carried out using tissue directly obtained from samples of infected fish in fish farm cages. The tissue samples were from intestine, spleen, liver, kidney and brain. A portion of the tissue was cut to obtain the tissue sample (≤ 20 mg), which was aseptically homogenized in small pieces to proceed with DNA extraction as described before. PCR amplification of each tissue sample of 16S rDNA gene was performed, as well as the above described subsequent enzymatic digestion.

### Example 9: Piscirickettsia salmonis detection:

Figure 4 shows the curve of bacterial growth, starting with an inoculum with an OD₆₀₀ = 0.05. The exponential phase takes place between days two and seven as it was previously reported by Figueroa et al. 2012. To detect *Piscirickettsia salmonis* in the growth medium, the mentioned techniques described for this purpose were tested: microscopic visualization of the culture: Fryer, J. L., Lannan, C. N., Giovannoni, S. J. & Wood, N. D. Piscirickettsia salmonis gen. nov. sp. nov., the causative agent of an epizootic disease in salmonid fishes. Int. J. Syst. Bacteriol. 42, 120-6 1992; Lannan, C. N., Ewing, S. a. & Fryer, J. L. A Fluorescent Antibody Test for Detection of the Rickettsia Causing Disease in Chilean Salmonids. J. Aquat. Anim. Health 3, 229-234 1991. See Figures 3A and 3B and PCR based techniques, Mauel et al.1996; Karatas, S. et al. Real time PCR detection of Piscirickettsia salmonis from formalin-fixed paraffin-embedded tissues. J. Fish Dis. 31, 747-53 2008; see Figures 5C and 5D. In the methodology of visualization by Gram staining, a gram negative coccus is observed, see Figure 4A, while *Piscirickettsia salmonis* colony is grey, opaque and of a circular shape, see Figure 4A. In addition, DAPI staining, immunodetection with the commercially available antibody used as a reference against *Piscirickettsia salmonis* Lannan, C. N., Ewing, S. A. & Fryer, J. L. A Fluorescent Antibody Test for Detection of the Rickettsia Causing Disease in Chilean Salmonids. J. Aquat. Anim. Health 3, 229-234 1991 and the image of fused fluorescence signals is shown in Figure 3B. The fused images show the correlation between DAPI staining and the antibody localization, which allows identifying *Piscirickettsia salmonis* in the culture medium.

On the other hand, detection techniques that involve amplification by means of PCR with specific identification primers are shown in Figures 4C and 4D. Figure 4C corresponds to an electrophoresis in agarose gel of the amplification product obtained using universal primers (27F and 1492R) of bacterial 16S gene, and Nested-PCR with PsAs and Ps2s primers described in Mauel et al. 1996, identifies a 1547 bp fragment and a 469 bp fragment, respectively as described by the authors. Figure 5D shows the amplification and fusion curves of qPCR reactions that contain or do not contain *Piscirickettsia salmonis* DNA substrate using Ps16Sreal-F1/Ps16Sreal-R primers, Karatas, S. et al. Real time PCR detection of Piscirickettsia salmonis from formalin-fixed paraffin-embedded tissues. J. Fish Dis. 31, 747-53 2008. The amplification of *Piscirickettsia salmonis* is initiated in cycle 10.59, and the analysis of the fusion curve shows a single peak for the bacterium.

### Example 10: Bacterial Strain Classification and Philogenetics

After isolation, the bacteria were classified into three categories: fish pathogen, fish isolate and laboratory contaminant, see Table 2. The sequence obtained by ribosomal DNA amplification was used as a template to search in RDP databases data to assign sequence identification.

**Table 2: Strains used. Twelve strains were identified according to the best match obtained when searching in the Ribosomal Database Project RDP. Sequence identity score between 0 and 1.**

| **RDP Code** | **Score** | **Best Match** | **Category** | **Reference** |
|---|---|---|---|---|
| S003803882 | 0.991 | *Pisciricketssia salmonis* | Fish pathogen | Bravo et al 2012 |
| S003807761 | 1.000 | *Vibrio angullarum* | Fish pathogen | Frans. et al 2013 |
| S000494055 | 1.000 | *Aeromonas salmonicida* | Fish pathogen | Haveman S. A. et al 2005 |
| S002288664 | 0.876 | *Flavobacterium psychrophilum* | Fish pathogen | Duchaud et al 2007 |
| S000487822 | 0.895 | *Renibacterium salmoninarum* | Fish pathogen | Konigsson et al 2005 |
| S000134127 | 0.996 | *Shewanella frigidimarina* | Isolate from fish | Bozal N et al 2001 |
| S000407844 | 1.000 | *Photobacterium phosphoreum* | Isolate from fish | Budsberg K J et al 2003 |
| S003721215 | 0.995 | *Psychrobacter sp* | Isolate from fish | Xing M et al 2013 |
| S001589700 | 0.995 | *Arthobacter sp* | Laboratory contaminant | Mou et al 2009 |
| S003715369 | 0.998 | *Staphylococcus saprophyticus* | Laboratory contaminant | Zhang X et al 2012 |
| S000842579 | 0.981 | *Micobacterium aurum* | Laboratory contaminant | Dekas A et al 2006 |
| S001572565 | 1.000 | *Escherichia coli* | Laboratory contaminant | Yoshiyama M et al 2009 |

### Example 11: Analysis of the Specificity of existing techniques for Piscirickettsia salmonis identification

In order to determine the specificity of the diagnostic techniques commonly used to detect *Piscirickettsia salmonis,* some assays were conducted using the bacteria described in Table 2.

The first assay evaluated the use of the commercial antibody against *Piscirickettsia salmonis* BioSigma to support Piscirickettsia *salmonis* detection in culture samples, in accordance with the indications recommended by the manufacturer. By means of confocal microscopy, *Piscirickettsia salmonis* immunodetection was detected and a similar match of antibody signals in two marines isolates corresponding to the genus *Shewanella* and *Photobacterium* (Figure 9), this being considered a non-specific immunodetection. For all the other strains, the tested antibody was negative.

On the other hand, Nested PCR, Mauel et al. 1996, described as specific for *Piscirickettsia salmonis,* was evaluated in all the contaminant strains (Table 2). The results indicate that besides *Piscirickettsia salmonis,* eight isolated bacteria show the described band of 469 bp as specific, as well as other non-specific amplicons (Figure 5). In addition, the specific reaction of quantitative PCR of *Piscirickettsia salmonis* using 16SRNA-F1/16SRNA-R, resulted in the amplification of all the contaminant strains, with the exception of *Microbacterium aurum* and *Renibacterium salmoninarum.* Although in this assay the cycle threshold values (Ct), obtained with *Piscirickettsia salmonis* DNA corresponded to early cycles, ADN amplification was observed in other bacteria at later cycles, and in all cases melting curves were produced ("melting curves") with non-significant differences in the values of melting temperature (Mt), this did not allow to distinguish *Piscirickettsia salmonis* specifically from other analysed bacteria, see Table 3. In the same manner, when testing a Taqman amplification probe, described as specific for *Piscirickettsia salmonis,* Corbeil, S., Mccoll, K. A. & Crane, M. S. J. Development of a TaqMan quantitative PCR assay for the identification of Piscirickettsia salmonis. Bull. Eur. Assoc. Fish Pathol. 23, 95-101 2000, the assay resulted positive for all the samples in Table 3, implying that the probe lacks specificity for *Piscirickettsia salmonis.*

**Table 3: Assays based on quantitative PCR. qPCR based on 16SRNA-F1/16SRNA-R primers, Karatas, S. et al. Real time PCR detection of Piscirickettsia salmonis from formalin-fixed paraffin-embedded tissues. J. Fish Dis. 31, 747-53 2008. Ct amplification cycle, Mt melting temperature, EGC equivalent genome copies and status of the samples obtained for each assay during 16S ribosomal DNA amplification. *Indicates values that are significantly different. One way ANOVA p>0,005.**

| **Sample** | **Qpcr Assay** | | | **Taqman Assay** | | |
|---|---|---|---|---|---|---|
| | Ct | Mt | Sample status | Ct | EGC | Sample status |
| *Pisciricketssia salmonis* | 10.59±0.24* | 89.4±0.0 | positive | 11.2 | 7.8x10¹⁰ | positive |
| *Vibrio angullarum* | 18.73±0.89 | 87.3±1.48 | positive | 24.8 | 4.9x10⁶ | positive |
| *Aeromonas salmonicida* | 22.31±1.35 | 91.5±1.09 | positive | 23.7 | 1.0x10⁷ | positive |
| *Flavobacterium psychrophilum* | 20.21±0.35 | 89.0±0.33 | positive | 21.3 | 6.0x10⁷ | positive |
| *Renibacterium salmoninarum* | - | - | negative | 31.8 | 8.0x10³ | positive |
| *Shewanella frigidimarina* | 21.33±0.79 | 87.8±0.47 | positive | 28.1 | 3.0x10⁵ | positive |
| *Photobacterium phosphoreum* | 25.16±1.02 | 88.8±0.30 | positive | 27.3 | 6.5x10⁵ | positive |
| *Psychrobacter sp* | 22.19±0.93 | 87.7±0.59 | positive | 28.5 | 2.0x10⁵ | positive |
| *0Arthobcter sp* | 27.68±0.84 | 92.3±0.03 | positive | 29.0 | 1.3x10⁵ | positive |
| *Staphylococcus saprophyticus* | 25.39±0.84 | 89.4±0.09 | positive | 28.8 | 1.6x10⁵ | positive |
| *Micobacterium aurum* | - | - | negative | 27.4 | 5.7x10⁵ | positive |
| *Escherichia coli* | 23.93±1.92 | 89.0±0.31 | positive | 35.8 | 6.8x10¹ | positive |

### Example 12: Example of application group I

### Example 12 A: Application in a sample infected with Piscirickettsia salmonis

At least 200 mg of tissue are obtained from the infected specimen; then, bacterial DNA extraction from the tissue sample is carried out using conventional techniques and according to the instructions of the manufacturer in the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme PmlI. The electrophoretic bands or the digestion pattern of the product are compared to a DNA standard (ladder) and to the first pattern of Figure 2 that is characteristic of *Piscirickettsia salmonis,* and there is a 100% match; then the presence of *Piscirickettsia salmonis* is confirmed in the sample.

The above because the pattern exhibits electrophoretic bands of 733 bp, 396 bp, 283 bp, and 97 bp.

### Example 12 B: Application in a sample infected by a pathogen different from Piscirickettsia salmonis (Staphylococcus saprophyticus).

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme PmlI. The electrophoretic bands or the digestion pattern of the product are compared to a DNA standard (ladder) and to the first pattern of Figure 2 that is characteristic of *Piscirickettsia salmonis* and there is not a 100% match; then it is confirmed that *Piscirickettsia salmonis* is not present in the sample.

The above because the pattern exhibits electrophoretic bands of 1118 bp, 284 bp and 114 bp. These results discard that the infection of the sample is produced by *Piscirickettsia salmonis*

### Example 12 C: Application in an infected sample treated with a restriction enzyme that does not allow discriminating Piscirickettsia salmonis from other bacteria causing the infection.

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme TaqI. The electrophoretic bands or the digestion pattern of the product are compared to a DNA standard (ladder) and to the first pattern of Figure 2 that is characteristic of *Piscirickettsia salmonis* and there is not a 100% match; then it is not possible to determine the pathogen that is infecting the sample since a unique pattern is not available because the enzyme exhibits the electrophoretic bands of the bp indicated below in Table 4.

**Table 4**

| **Bacteria** | | **Confusing Pattern 1 (TaqI) pb** | | | | | | |
|---|---|---|---|---|---|---|---|---|
| *Piscirickettsia salmonis* | | 901 | 361 | 192 | 55 | | | |
| *Vibrio anguillarum* | | 899 | 361 | 193 | 55 | | | |
| *Aeromonas salmonicida* | | 683 | 361 | 194 | 86 | 80 | 55 | 53 |
| *Flavobacterium psychrophilum* | | 883 | 316 | 184 | 55 | 41 | | |
| *Renibacterium salmoninarum* | | 891 | 360 | 147 | 55 | 50 | | |
| *Shewanella frigidimarina* | | 901 | 361 | 220 | 55 | | | |
| *Photobacterium phosphoreum* | | 906 | 359 | 194 | 55 | | | |
| *Psychrobacter sp.* | | 597 | 361 | 198 | 198 | 66 | 55 | 34 |
| *Arthrobacter sp.* | | 878 | 359 | 148 | 55 | 50 | | |
| *Staphylococcus saprophyticus* | | 907 | 361 | 145 | 55 | 48 | | |
| *Microbacterium aurum* | | 664 | 360 | 146 | 120 | 95 | 55 | 50 |
| *Escherichia coli* | | 759 | 360 | 221 | 86 | 55 | 53 | |

### Example 13: Example of application group II

### Example 13 A: Application in a sample infected with Piscirickettsia salmonis

At least 200 mg of tissue are obtained from the infected specimen; then, bacterial DNA extraction from the tissue sample is carried out using conventional techniques and according to the instructions of the manufacturer of the kit to be used. Afterwards the purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme BspCNI. The electrophoretic bands or the digestion pattern of the product are compared to a DNA standard (ladder) and to the second pattern of Figure 2 that is characteristic of *Piscirickettsia salmonis,* and there is a 100% match; then the presence of *Piscirickettsia salmonis* is confirmed in the sample.

The above because the pattern exhibits electrophoretic bands of 529 bp, 434 bp, 239 bp, 140 bp, 138 bp and 29 bp.

### Example 13B: Application in sample infected by a pathogen different from Piscirickettsia salmonis (Renibacterium salmoninarum)

At least 200 mg of tissue are obtained from the infected specimen; then, bacterial DNA extraction from the tissue sample is carried out using conventional techniques and according to the instructions of the manufacturer of the kit to be used. Afterwards, the purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme BspCNI. The electrophoretic bands or the digestion pattern of the product are compared to a DNA standard (ladder) and to the second pattern of Figure 2 that is characteristic of *Piscirickettsia salmonis,* and there is not a 100% match; then it is confirmed that *Piscirickettsia salmonis* is not present in the sample.

The above because the pattern exhibits electrophoretic bands of 563 bp, 426 bp, 249 bp, 209 bp, 29 bp and 27 bp. These results discard that the sample infection is produced by *Piscirickettsia salmonis.*

### Example 13C: Application in an infected sample treated with a restriction enzyme that does not allow discriminating Piscirickettsia salmonis from other bacteria causing the infection.

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme RsaI. The electrophoretic bands or the digestion pattern of the product are compared to a DNA standard (ladder) and to the second pattern of Figure 2 that is characteristic of *Piscirickettsia salmonis* and there is not a 100% match; then it is not possible to determine the pathogen that is infecting the sample because a unique pattern is not available since the enzyme exhibits electrophoretic bands of the bp indicated below in Table 5.

**Table 5**

| **Bacteria** | | **Confusing Pattern 2 (RsaI) pb** | | | | | | | | |
|---|---|---|---|---|---|---|---|---|---|---|
| *Piscirickettsia salmonis* | | 886 | 357 | 146 | 120 | | | | | |
| *Vibrio anguillarum* | | 650 | 503 | 234 | 121 | | | | | |
| *Aeromonas salmonicida* | | 887 | 357 | 146 | 122 | | | | | |
| *Flavobacterium psychrophilum* | | 400 | 354 | 161 | 146 | 126 | 98 | 83 | 77 | 34 |
| *Renibacterium salmoninarum* | | 469 | 252 | 227 | 155 | 147 | 129 | 121 | 3 | |
| *Shewanella frigidimarina* | | 886 | 357 | 148 | 146 | | | | | |
| *Photobacterium phosphoreum* | | 457 | 434 | 399 | 122 | 102 | | | | |
| *Psychrobacter sp.* | | 880 | 357 | 146 | 126 | | | | | |
| *Arthrobacter sp.* | | 456 | 355 | 252 | 155 | 147 | 122 | 3 | | |
| *Staphylococcus saprophyticus* | | 503 | 486 | 406 | 121 | | | | | |
| *Microbacterium aurum* | | 457 | 356 | 252 | 155 | 147 | 120 | 3 | | |
| *Escherichia coli* | | 502 | 456 | 427 | 149 | | | | | |

### Example 14: Example application group III

### Example 14A: Application in a sample infected with Piscirickettsia salmonis

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme SSe9I. The electrophoretic bands or the digestion pattern of the product are compared to a DNA standard (ladder) and to the third pattern of Figure 2 that is characteristic of *Piscirickettsia salmonis,* and there is a 100% match; then it is confirmed that *Piscirickettsia salmonis* is present in the sample.

The above because the pattern exhibits electrophoretic bands of 419 bp, 334 bp, 245 bp, 119 bp, 116 bp, 92 bp, 82 bp, 46 bp, 40 bp and 16 bp.

### Example 14B: Application in a sample infected by a pathogen different from Piscirickettsia salmonis (Photobacterium phosphoreum)

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme Sse9I. The electrophoretic bands or the digestion pattern of the product are compared to a DNA standard (ladder) and to the third pattern of Figure 2 that is characteristic of *Piscirickettsia salmonis,* and there is not a 100% match; then it is confirmed that *Piscirickettsia salmonis* is not present in the sample.

The above because the pattern exhibits electrophoretic bands of 558 bp, 557 bp, 249 bp, 116 bp and 40 bp. These results discard that the sample infection is produced by *Piscirickettsia salmonis.*

### Example 14C: Application in an infected sample treated with a restriction enzyme that does not allow discriminating Piscirickettsia salmonis from other bacteria causing the infection.

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme SmII. The electrophoretic bands or the digestion pattern of the product are compared to a DNA standard (ladder) and to the third pattern of Figure 2 that is characteristic of *Piscirickettsia salmonis,* and there is not a 100% match; then it is not possible to determine the pathogen that is infecting the sample since a unique pattern is not available as the enzyme exhibits electrophoretic bands of the bp indicated below in Table 6.

**Table 6**

| **Bacteria** | | **Confusing Pattern 3 (SmlI) pb** | | |
|---|---|---|---|---|
| *Piscirickettsia salmonis* | | 835 | 674 | |
| *Vibrio anguillarum* | | 834 | 674 | |
| *Aeromonas salmonicida* | | 837 | 368 | 307 |
| *Flavobacterium psychrophilum* | | 1479 | | |
| *Renibacterium salmoninarum* | | 1503 | | |
| *Shewanella frigidimarina* | | 836 | 701 | |
| *Photobacterium phosphoreum* | | 841 | 673 | |
| *Psychrobacter sp.* | | 830 | 609 | 70 |
| *Arthrobacter sp.* | | 1490 | | |
| *Staphylococcus saprophyticus* | | 651 | 466 | 399 |
| *Microbacterium aurum* | | 1490 | | |
| *Escherichia coli* | | 701 | 644 | 189 |

### Example 15: Example application group IV

### Example 15A: Application in sample infected with Piscirickettsia salmonis

At least 200 mg of tissue are obtained from the infected specimen; then bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme XspI. The electrophoretic bands or the digestion pattern of the product are compared to a DNA standard (ladder) and to the fourth pattern of Figure 2 that is characteristic of *Piscirickettsia salmonis,* and there is a 100% match; then the presence of *Piscirickettsia salmonis* is confirmed in the sample.

The above because the pattern exhibits: electrophoretic bands of 409 bp, 326 bp, 195 bp, 175 bp, 135 bp, 95 bp, 74 bp, 71 bp, 27 bp and 8 bp.

### Example 15B: Application in a sample infected by a pathogen different from Piscirickettsia salmonis (Microbacterium aurum)

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme XspI. The electrophoretic bands or the digestion pattern of the product are compared to a DNA standard (ladder) and to the fourth pattern of Figure 2 that is characteristic of *Piscirickettsia salmonis,* and there is not a 100% match; then it is confirmed that *Piscirickettsia salmonis* is not present in the sample.

The above because the pattern exhibits: electrophoretic bands of 622 bp, 520 bp, 175 bp and 173 bp. These results discard that the sample infection is produced by *Piscirickettsia salmonis.*

### Example 15C. Application in an infected sample treated with a restriction enzyme that does not allow discriminating Piscirickettsia salmonis from other bacteria causing the infection.

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme BaeI. The electrophoretic bands or the digestion pattern of the product are compared to a DNA standard (ladder) and to the fourth pattern of Figure 2 that is characteristic of *Piscirickettsia salmonis,* and there is not a 100% match; then it is not possible to determine the pathogen that is infecting the sample since a unique pattern is not available as the enzyme exhibits the electrophoretic bands of the bp indicated below in Table 7.

**Table 7**

| **Bacteria** | | **Confusing Pattern 4 (BaeI) pb** | | | | |
|---|---|---|---|---|---|---|
| *Piscirickettsia salmonis* | | 776 | 700 | 33 | | |
| *Vibrio anguillarum* | | 776 | 699 | 33 | | |
| *Aeromonas salmonicida* | | 700 | 529 | 217 | 33 | 33 |
| *Flavobacterium psychrophilum* | | 765 | 681 | 33 | | |
| *Renibacterium salmoninarum* | | 766 | 704 | 33 | | |
| *Shewanella frigidimarina* | | 778 | 726 | 33 | | |
| *Photobacterium phosphoreum* | | 783 | 698 | 33 | | |
| *Psychrobacter sp.* | | 772 | 480 | 191 | 33 | 33 |
| *Arthrobacter sp.* | | 753 | 704 | 33 | | |
| *Staphylococcus saprophyticus* | | 783 | 700 | 33 | | |
| *Microbacterium aurum* | | 754 | 703 | 33 | | |
| *Escherichia coli* | | 775 | 726 | 33 | | |

### Example 16: Example of application group V

### Example 16A: Application in sample infected with Piscirickettsia salmonis

At least 200 mg of tissue are obtained from the infected specimen; then, bacterial DNA extraction from the tissue sample is carried out using conventional techniques and according to the instructions of the manufacturer in the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme DdeI. The electrophoretic bands or the digestion pattern of the product are compared to a DNA standard (ladder) and to the fifth pattern of Figure 2 that is characteristic of *Piscirickettsia salmonis,* and there is a 100% match; then the presence of *Piscirickettsia salmonis* is confirmed in the sample.

The above because the pattern exhibits electrophoretic bands of 337 bp, 245 bp, 231 bp, 163 bp, 161 bp, 138 bp, 97 bp, 83 bp, 38 bp and 16 bp.

### Example 16 B: Application in a sample infected by a pathogen different from Piscirickettsia salmonis (Aeromonas salmonicida)

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme DdeI. The electrophoretic bands or the digestion pattern of the product are compared to a DNA standard (ladder) and to the fifth pattern of Figure 2 that is characteristic of *Piscirickettsia salmonis,* and there is not a 100% match; then it is confirmed that *Piscirickettsia salmonis* is not present in the sample.

The above because the pattern exhibits electrophoretic bands of 760 bp, 434 bp, 275 bp, 27 bp and 16 bp. These results discard that the sample infection is produced by *Piscirickettsia salmonis.*

### Example 16C: Application in an infected sample treated with a restriction enzyme that does not allow discriminating Piscirickettsia salmonis from other bacteria causing the infection.

At least 200 mg of tissue are obtained from the infected specimen; then, bacterial DNA extraction from the tissue sample is carried out using conventional techniques and according to the instructions of the manufacturer of the kit to be used. Afterwards, purity of the total isolated bacterial DNA is verified using conventional techniques, for example, spectrophotometry.

The amplification of the extracted DNA is performed by PCR using 27F 5' AGA GTT TGA TCA TGG CTC AG 3' and 1492R 5' TAC GGT TAC CTT GTT ACG ACT T 3' primers; the product is digested using restriction enzyme SmaI. The electrophoretic bands or the digestion pattern of the product are compared to a DNA standard (ladder) and to the fourth pattern of Figure 2 that is characteristic of *Piscirickettsia salmonis,* and there is not a 100% match; then it is not possible to determine the pathogen that is infecting the sample since a unique pattern is not available as the enzyme exhibits the electrophoretic bands of the bp indicated below in Table 8.

**Tabla 8**

| **Bacteria** | | **Confusing Pattern 5 (SmaI) pb** | | |
|---|---|---|---|---|
| *Piscirickettsia salmonis* | | 1380 | 129 | |
| *Vibrio anguillarum* | | 772 | 606 | 130 |
| *Aeromonas salmonicida* | | 771 | 610 | 131 |
| *Flavobacterium psychrophilum* | | 1359 | 120 | |
| *Renibacterium salmoninarum* | | 1370 | 133 | |
| *Shewanella frigidimarina* | | 771 | 609 | 157 |
| *Photobacterium phosphoreum* | | 770 | 613 | 131 |
| *Psychrobacter sp.* | | 771 | 603 | 135 |
| *Arthrobacter sp.* | | 1356 | 134 | |
| *Staphylococcus saprophyticus* | | 1386 | 130 | |
| *Microbacterium aurum* | | 1358 | 132 | |
| *Escheric hia coli* | | 769 | 607 | 158 |

*Piscirickettsia salmonis* is an important etiologic agent for a fish disease that causes great economic losses in the salmon industry. For this reason, several efforts have been made in order to develop an epidemiologic control of this bacterium that is responsible of the SRS syndrome.

In the Chilean industry, vaccines have been an important approach to decrease fish mortality and reduce the need of medical treatment for various bacterial diseases in farmed fish. However, in the case of SRS the vaccines used have not shown a significant reduction in fish mortality: Bravo, S. & Midtlyng, P. J. The use of fish vaccines in the Chilean salmon industry 1999-2003. Aquaculture 270, 36-42 2007. For this reason, the correct identification as well as the confirmation of *Piscirickettsia salmonis* purity in culture media and tissue samples has become critical. Then, the PCR-RFLP method has turned out to be an important tool to attain this purpose as it is based on SNP dependent patterns and this genetic characteristic allows determining purity.

The present method allows to certify and ascertain the purity of *Piscirickettsia salmonis* culture samples as well as to identify it in infected tissues. This method also improves the specificity of the other tested techniques in a simple and rapid way. It makes possible to differentiate it among all the bacteria that present the greatest change on cohabiting with *Piscirickettsia salmonids.*

## Claims

1. A rapid, specific, sensitive, low cost and reproducible method for detecting the presence of *Piscirickettsia salmonis* in a sample, wherein it comprises amplifying a fragment of 16S ribosomal gene shared by all the bacteria present in a tissue sample, a bacterial culture and a cell culture (host) and subsequently digesting the amplified by the individual or combined use of restriction enzymes (PCR-RFLP) selected from 5 isoschizomer groups, wherein the first group comprises restriction enzymes PmII, PmaCI, AcvI, BbrPI, Eco72I or PspCI, all of them independently having cutting sequence CAC/GTG; the second group comprises enzymes selected from BspCNI or BseMII, all of them independently having cutting sequence CTCAG9/7; the third group comprises enzymes selected from TspEI, MluCI, Sse9I, TasI or Tsp509I, all of them independently having cutting sequence AATT; the fourth group comprises enzymes selected from BfaI, FspBI, XspI or MaeI, all of them independently having cutting sequence C/ATG; and the fifth group comprises enzymes selected from DdeI, BstDEI or HpyF3I, all of them independently having cutting sequence C/TNAG; which recognize exclusive nucleotide sequences in the 16S rDNA gene of *Piscirickettsia salmonis;* and after having obtained the electrophoretic bands or digestion patterns of the PCR product, comparing the bands obtained against any one or all the 5 electrophoretic bands or specific digestion patterns of Figure 2 that are characteristic of *Piscirickettsia salmonis;* a mismatch between the digestion pattern obtained from the sample and any one of the digestion patterns of Figure 2 indicates that *Piscirickettsia salmonis* is not present or the contamination or presence of other bacteria in the sample, while the match between the digestion pattern obtained from the sample with any one of the digestion patterns of Figure 2, indicates the presence of *Piscirickettsia salmonis* in the sample.

2. The method of claim 1, wherein it further allows to analyse *Piscirickettsia salmonis* cultures certifying purity thereof.

3. The method of claim 1, wherein the sample is selected from the group that consists of infected salmon tissue, a cell culture infected with *Piscirickettsia salmonis, Piscirickettsia salmonis* growing in an artificial medium in a bacterial culture or tissue that is directly obtained from samples that were obtained, in turn, from infected fish in the cages.

4. The method of claim 1, wherein the tissue samples are obtained from tissue of the intestine, spleen, liver, kidney and brain of the fish.

5. The method of claim 1, wherein it comprises the amplification with primers selected from 27F 5' AGA GTT TGA TCA TGG CTC AG 3' and 1492R 5' TAC GGT TAC CTT GTT ACG ACT T 3'.

6. The method of claim 1, wherein the restriction enzyme is selected from the group comprising PmII, PmaCI, AcvI, BbrPI, Eco72I or PspCI, all of them independently having cutting sequence CAC/GTG.

7. The method of claim 1, wherein the restriction enzyme is selected from the group comprising BspCNI or BseMII, all of them independently having cutting sequence CTCAG9/7.

8. The method of claim 1, wherein the restriction enzyme is selected from the group comprising TspEI, MluCI, Sse9I, TasI or Tsp509I, all of them independently having cutting sequence AATT.

9. The method of claim 1, wherein the restriction enzyme is selected from the group comprising BfaI, FspBI, XspI or MaeI, all of them independently having cutting sequence C/ATG.

10. The method of claim 1, wherein the restriction enzyme is selected from the group comprising DdeI, BstDEI or HpyF3I, all of them independently having cutting sequence C/TNAG.

11. A kit for detecting the presence of *Piscirickettsia salmonis* in a sample, wherein it comprises the components for characterizing at least one of the 5 specific digestion patterns of Figure 2 that are characteristic of *Piscirickettsia salmonis,* primers for the amplification of 16S ribosomal gene of *Piscirickettsia salmonis* selected from 27F 5' AGA GTT TGA TCA TGG CTC AG 3' or 1492R 5' TAC GGT TAC CTT GTT ACG ACT T 3', and at least one restriction enzyme selected from 5 different endonuclease groups that share the same cutting sequence, wherein the first group comprises restriction enzymes selected from PmII, PmaCI, AcvI, BbrPI, Eco72I or PspCI, all of them independently having cutting sequence CAC/GTG; the second group comprises enzymes selected from BspCNI or BseMII, all of them independently having cutting sequence CTCAG9/7; the third group comprises enzymes selected from TspEI, MluCI, Sse9I, TasI or Tsp509I, all of them independently having cutting sequence AATT; the fourth group comprises enzymes selected from BfaI, FspBI, XspI or MaeI, all of them independently having cutting sequence C/ATG; and the fifth group comprises enzymes selected from DdeI, BstDEI or HpyF3I, all of them independently having cutting sequence C/TNAG; which recognize the sequences exclusively identified in the 16S rDNA gene de *Piscirickettsia salmonis.*

12. The kit of claim 11, wherein it comprises the digestion pattern associated to the first group that comprises restriction enzymes selected from PmII, PmaCI, AcvI, BbrPI, Eco72I or PspCI, all of them independently having cutting sequence CAC/GTG of Figure 2, primers for the amplification of the 16S ribosomal gene of *Piscirickettsia salmonis,* and at least one restriction enzyme selected from the first group that comprises restriction enzymes selected from PmII, PmaCI, AcvI, BbrPI, Eco72I o PspCI, all of them independently having cutting sequence CAC/GTG.

13. The kit of claim 11, wherein it comprises the digestion pattern associated to the first group that comprises restriction enzymes selected from BspCNI o BseMII, all of them independently having cutting sequence CTCAG9/7 of Figure 2, primers for the amplification of the 16S ribosomal gene of *Piscirickettsia salmonis,* and at least one restriction enzyme selected from the first group that comprises restriction enzymes selected from BspCNI or BseMII, all of them independently having cutting sequence CTCAG9/7.

14. The kit of claim 11, wherein it comprises the digestion pattern associated to the first group that comprises restriction enzymes selected from TspEI, MluCI, Sse9I, TasI o Tsp509I, all of them independently having cutting sequence AATT de la Figure 2, primers for the amplification of the 16S ribosomal gene of *Piscirickettsia salmonis,* and at least one restriction enzyme selected from the first group that comprises restriction enzymes selected from BspCNI or BseMII, all of them independently having cutting sequence AATT.

15. The kit of claim 11, wherein it comprises the digestion pattern associated to the first group that comprises restriction enzymes selected from BfaI, FspBI, XspI o MaeI, all of them independently having cutting sequence C/ATG of Figure 2, primers for the amplification of the 16S ribosomal gene of *Piscirickettsia salmonis,* and at least one restriction enzyme selected from the first group that comprises restriction enzymes selected from BfaI, FspBI, XspI or MaeI, all of them independently having cutting sequence C/ATG.

16. The kit of claim 11, wherein it comprises the digestion pattern associated to the first group that comprises restriction enzymes selected from DdeI, BstDEI or HpyF3I, all of them independently having cutting sequence CAC/GTG of Figure 2, primers for the amplification of the 16S ribosomal gene of *Piscirickettsia salmonis,* and at least one restriction enzyme selected from the first group that comprises restriction enzymes selected from DdeI, BstDEI or HpyF3I, all of them independently having cutting sequence CAC/GTG.
